# EUROPEAN PATENT SPECIFICATION

(11) **EP 3 464 585 B1**
(45) Date of publication and mention of the grant of the patent: **11.03.2020**
(21) Application number: 17730677.6
(22) Date of filing: 19.05.2017
(51) Int. Cl.: C12N 11/00, C08L 1/00, C08L 3/00, C08L 5/00, C08L 23/00, C08L 33/12, C08L 33/26, C08L 71/02, C08L 77/04, C08L 79/02

(54) **AZLACTONE FUNCTIONALIZED SUBSTRATES FOR CONJUGATION OF BIOMOLECULES**
AZLACTONEFUNKTIONALISIERTE SUBSTRATE ZUR KONJUGATION VON BIOMOLEKÜLEN
SUBSTRATS FONCTIONNALISÉS D'AZLACTONE POUR LA CONJUGATION DE BIOMOLÉCULES

(30) Priority: 31.05.2016 US 201615169008
(43) Date of publication of application: 10.04.2019
(73) Proprietor: Robert Bosch GmbH, 70442 Stuttgart (DE)
(72) Inventor: MARUNIAK, Autumn, Fremont, California 94536 (US); AHMAD, Habib, Sunnyvale, California 94086 (US); JOHNSON, Christopher, San Carlos, California 94070 (US); KAVUSI, Sam, Menlo Park, California 94025 (US); FOMINA, Nadezda, Fremont, California 94555 (US)
(86) International application number: PCT/EP2017/062083
(87) International publication number: WO 2017/207298

(56) References cited:
- WO-A1-94/18247
- TIMOTHY C. LOGAN ET AL: "Photopatterning Enzymes on Polymer Monoliths in Microfluidic Devices for Steady-State Kinetic Analysis and Spatially Separated Multi-Enzyme Reactions", ANALYTICAL CHEMISTRY, vol. 79, no. 17, 1 September 2007 (2007-09-01), pages 6592-6598, XP055407199, US ISSN: 0003-2700, DOI: 10.1021/ac070705k
- MAREN E. BUCK ET AL: "Azlactone-functionalized polymers as reactive platforms for the design of advanced materials: Progress in the last ten years", POLYMER CHEMISTRY, vol. 3, no. 1, 1 January 2012 (2012-01-01) , pages 66-80, XP055407010, GB ISSN: 1759-9954, DOI: 10.1039/C1PY00314C
- GREGOIRE CARDOEN ET AL: "Synthesis of heterobifunctional polyethylene glycols with azide functionality suitable for "click" chemistry", JOURNAL OF POLYMER RESEARCH, KLUWER ACADEMIC PUBLISHERS-CONSULTANTS BUREAU, NL, vol. 19, no. 4, 3 April 2012 (2012-04-03), pages 1-10, XP035045087, ISSN: 1572-8935, DOI: 10.1007/S10965-012-9856-Y

## Description

### FIELD OF THE INVENTION

Aspects of the present invention relate to a bifunctional polymer and methods of making the bifunctional polymer, where the bifunctional polymer is functionalized at one end with an azlactone group to conjugate biomolecules of interest, and functionalized at another end with an azide anchor group to attach the polymer to a substrate, such that azlactone functionality remains intact under conditions where the azide group is reactive. Aspects of the present invention also relate to a coated substrate that includes such bifunctionalized polymers on the surface of the substrate, and methods of making the coated substrate. Aspects of the present invention also relate to a microarray that includes a plurality of discrete spots, each spot including the coated substrate on the surface of the microarray.

### BACKGROUND

In measurement assays or platforms, such as protein and antibody microarrays, achieving high fidelity (e.g., sensitivity and specificity) of the target molecule is of great importance. The samples in assays can be biomolecules such as nucleic acids, proteins, biological cells, and small molecules, along with other human bodily fluids such as blood, serum, saliva, and urine, and also consumables such as milk, baby food, or water. Regardless of the sample, efficient conjugation of the target molecule to the substrate of the assay can help achieve higher fidelity in these assays.

Activated esters (N-hydroxysuccinimide (NHS), maleimide, fluorophenyl), carbamates, carbonates, epoxides, and aldehydes are functional groups that are commonly used in assays for conjugation of biomolecules. However, these functional groups suffer from low hydrolytic stability. Low hydrolytic stability can lead to suboptimal efficiency of conjugation of the biomolecules due to the competing hydrolysis reaction, which results in a lower amount of conjugated biomolecules and non-uniformity of conjugated biomolecules.

Azlactone groups are known to react with strong nucleophiles, such as primary amines, alcohols, and thiols, via a ring-opening addition reaction, while showing good resistance to water hydrolysis at a neutral pH (see Carter, H.E., Chapter 5: "Azlactones," Organic Reactions, John Wiley & Sons, 3:198-239 (1946)). In addition to higher stability, azlactone-functionalized surfaces can be stored longer under ambient conditions, while surfaces containing activated esters, epoxides, and aldehydes have to be stored in moisture-free conditions (vacuum-sealed packaging, freezer) and utilized immediately upon exposure to ambient conditions.

"Azlactone" can be represented by a 6-membered ring: 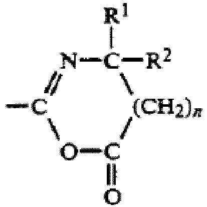 where R₁ and R₂ independently can be hydrogen, an alkyl group having 1 to 14 carbon atoms, a cycloalkyl group having 3 to 14 carbon atoms, an aryl group having 5 to 12 ring atoms, an arenyl group having 6 to 26 carbon atoms and 0 to 3 sulfur, nitrogen, and nonperoxidic oxygen heteroatoms, or R₁ and R₂ taken together with the carbon to which they are joined can form a carbo-cyclic ring containing 4 to 12 ring atoms; and n is an integer 0 or 1.

If n is 0, then azlactone can be represented by a 5-membered ring: 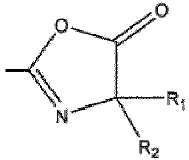 where R₁ and R₂ are defined as above.

Due to their advantageous and unique properties, methods of preparing azlactone functionalized polymers for use in protein conjugation are known. U.S. Pat. No. 5,321,095 discloses a method of producing azlactone-activated polyalkylene oxides, including polyethylene glycol (PEG), for the purpose of preparing protein-PEG conjugates. U.S. Pat. Nos. 4,485,236 and 5,013,795 also disclose methods of preparing azlactone-containing polymers via radical processes and their use for protein conjugation.

Azlactone groups have been utilized in chromatography. Rasmussen et al., "Crosslinked, hydrophilic, azlactone-functional polymeric beads: A two-step approach," Reactive Polymers, 16(2):199-212 (1991), discloses the application of azlactone-functionalized polymer beads for chromatography, where acrylamide-based polymer beads are produced via radical polymerization, and azlactone groups are introduced via cyclodehydration of pendant acylamino acid groups using acetic anhydride. Coleman et al., "Immobilization of Protein A at High Density on Azlactone-functional Polymeric Beads and Their Use in Affinity Chromatography," Journal of Chromatography, 512:345-63 (1990), discloses highly cross-linked copolymer beads with protein immobilized on their surfaces, prepared by an inverse-phase polymerization process from methylene-bis-acrylamide and vinyldimethyl azlactone, for use in affinity chromatography.

Azlactone groups have also been utilized in cell and enzyme immobilization substrates. Buck et al., "Chemical Modification of Reactive Multilayered Films Fabricated from Poly(2-alkenyl azlactone)s: Design of Surfaces That Prevent or Promote Mammalian Cell Adhesion and Bacterial Biofilm Growth," Biomacromolecules, 10(6): 1564-74 (2009), discloses reactive polymer films that can be functionalized to either prevent or promote an attachment and growth of cells through layer-by-layer assembly of polyamine and poly(2-vinyl-4,4'-dimethylazlactone) (PVDMA). The reaction between amino groups of polyamine with azlactone functionality of PVDMA results in covalent cross-linking of films. The unreacted azlactone groups of PVDMA are further utilized to attach hydrophilic or hydrophobic moieties, thus promoting either cell adhesion or cell repulsion from the surface. Cullen et al., "Surface-Anchored Poly(2-vinyl-4,4-dimethyl azlactone) Brushes as Templates for Enzyme Immobilization," Langmuir, 24(23): 13701-09 (2008), discloses growing PVDMA brushes from initiators anchored to the surface of glass via atom transfer radical polymerization.

Azlactone groups have also been utilized in medical implants. U.S. Pat. No. 5,292,514 discloses preparation of polymeric and oligomeric vinyldimethyl azlactones via radical polymerization and their use as coatings for mammalian body implants.

U.S. Pat. No. 4,981,933 discloses preparation of azlactone copolymers from unsaturated polymerizable azlactone polymer (such as vinyl azlactone) and vinylbenzylhalide, thus having the respective reaction functionalities of these two moieties.

U.S. Pat. No. 5,344,701 discloses several methods of introducing azlactone functionality onto existing supports for coupling of bioreagents. Those methods include high-energy radiation to generate free radicals that subsequently react with vinyl-azlactone, chemical crosslinking of azlactone monomers to form a film on the surface, and dispersion polymerization to produce functional particles within pores of the support.

### SUMMARY

A summary of certain example embodiments of the present invention is set forth below. It should be understood that these aspects are presented merely to provide the reader with a brief summary of these certain embodiments and that these aspects are not intended to limit the scope of the present invention. Indeed, this invention can encompass a variety of aspects that may not be set forth below.

Azlactone functionalized surfaces in measurement platforms or assays can potentially produce spots with superior brightness and uniformity. Because of higher stability of azlactone-functionalized surfaces to water hydrolysis, reproducibility of spotting can potentially be improved as well. But to utilize azlactone groups in measurement platforms or assays, the azlactone functionalized polymers should be immobilized to a surface or substrate without disrupting the functionality of the azlactone groups. In other words, a second functional group must attach the azlactone functionalized polymers to the surface or substrate, but should not interfere with the ability of the azlactone groups to conjugate biomolecules of interest.

Thus, example embodiments of the present invention provide a polymer functionalized with both azlactone groups to conjugate biomolecules and also attachment groups to immobilize the azlactone functionalized polymer onto a substrate to create a biocompatible surface, where azlactone functionality remains intact under the conditions that render the attachment group reactive. According to example embodiments, PEG is functionalized with azlactone groups for conjugation of biomolecules and with azide groups for attachment to a substrate. Azide and azlactone groups are chemically orthogonal, such that azlactone functionality will remain intact under the conditions that render the azide groups reactive. Example embodiments of the present invention also provide methods of making such biocompatible surfaces and incorporating such biocompatible surfaces as substrates in measurement platforms, such as protein and antibody micro arrays.

Example embodiments of the present invention provide azlactone functionalized polymers that can be anchored to a surface or substrate, where the resulting azlactone functionalized surface or substrate can be used in measurement assays, such as protein and antibody microarrays, for more efficient conjugation of biomolecules. Such functionalized surfaces can be utilized in biosensor systems as described in U.S. Pat. App. Ser. Nos. 14/792,553, 14/792,576, 14/792,541, 14/792,569, and 14/792,530, which are hereby incorporated herein by reference in their entireties.

According to example embodiments, a bifunctional polymer includes: (a) an anchor group selected from a group consisting of azide, carboxylic acid, thiol, amine, hydroxyl, hydrazine, silyl, phosphonate, alkyne, catechol, and lysine; (b) a polymer block that includes one or more first polymers, the one or more first polymers including PEG or polysaccharide; (c) a linker group selected from a group consisting of phenyl, vinyl, benzyl, and alkyl; and (d) an azlactone end group containing R₁ and R₂, where R₁ and R₂ are each independently selected from a group consisting of hydrogen, alkyl, and aryl.

In some example embodiments, the one or more first polymers are copolymers with a second polymer, where the second polymer is selected from a group consisting of polylysine, polyoxazoline, polymethylmethacrylate, poly-N-isopropylacrylamide, polydopamine, polyalkane, and N-substituted glycine polymer.

According to example embodiments, a coated substrate includes: (a) a substrate; and (b) a polymer layer attached to the substrate, where the polymer layer includes one or more first polymers, one or more azlactone functional groups attached to a first end of each of the one or more first polymers, and one or more azide groups attached to a second end of each of the one or more first polymers, the one or more azide groups attaching the polymer layer to the substrate.

In some example embodiments, the substrate is selected from a group consisting of glass, silica, plastic, carbon, metal, and metal oxide.

In some example embodiments, the polymer layer is linear polymer, multiarm polymer, brush polymer, or nanoparticles.

In some example embodiments, the one or more first polymers are PEG or polysaccharide.

In some example embodiments, the one or more first polymers are copolymers with a second polymer, where the second polymer is selected from a group consisting of polylysine, polyoxazoline, polymethylmethacrylate, poly-N-isopropylacrylamide, polydopamine, polyalkane, and N-substituted glycine polymer.

According to example embodiments, a microarray includes a plurality of discrete regions, where each discrete region includes a coated substrate, the coated substrate including: (a) a substrate; and (b) a polymer layer that is attached to the substrate and that includes one or more polymers, one or more azlactone functional groups attached to a first end of each of the one or more polymers and one or more azide groups attached to a second end of each of the one or more polymers, where the one or more azide groups attach the polymer layer to the substrate.

According to example embodiments, a method of preparing a bifunctional polymer includes: (a) providing a polymer with one or more carboxylic acid groups or activated ester groups attached to a first end of the polymer and one or more azide groups attached to a second end of the polymer; (b) converting at least one carboxylic acid group or activated ester group into an aryl or vinyl halide group; and (c) attaching vinyldialkyl azlactone to the aryl or vinyl halide group.

In some example embodiments, the polymer is PEG.

In some example embodiments, the vinyldialkyl azlactone is attached through a coupling reaction using a palladium catalyst, one or more solvents, and a base.

In some example embodiments, the palladium catalyst is selected from a group consisting of palladium (II) quinoline-8-carboxylate, palladium (II) chloride, palladium (II) bromide, palladium (II) acetate, palladium (II) acetoacetate, bis(dibenzylideneacetone)palladium(0), tris(dibenzylideneacetone)dipalladium(0), palladium (II) trifluoroacetate, allylpalladium (II) chloride dimer, bis(triphenylphosphine)palladium(II) dichloride, dichlorobis(tricyclohexylphosphine)palladium(II), and [1,1'-bis(di-tert-butylphosphino)ferrocene]dichloropalladium(II).

In some example embodiments, the one or more solvents are selected from a group consisting of dimethylformamide, dimethylsulfoxide, toluene, tetrahydrofuran, dioxane, dichloromethane, acetonitrile, and alcohol.

In some example embodiments, the base is selected from a group consisting of potassium carbonate, sodium carbonate, triethylamine, N,N-diisopropylethylamine, 4-(dimethylamino)pyridine, potassium tert-butoxide, and sodium tert-butoxide.

According to example embodiments, there is provided a method of attaching to a substrate a bifunctional polymer, the bifunctional polymer including a polymer, one or more azlactone functional groups attached to a first end of the polymer, and one or more azide groups attached to a second end of the polymer, the method including: (a) providing a substrate containing alkyne functional groups; (b) providing a mixture including the bifunctional polymer, one or more solvents, a catalyst, a base, and a reducing agent; and (c) contacting the substrate with the mixture.

In some example embodiments, the polymer is PEG.

In some example embodiments, the one or more solvents are selected from a group consisting of dimethylformamide, dimethylsulfoxide, toluene, tetrahydrofuran, dioxane, acetonitrile, and water.

In some example embodiments, the catalyst is a copper (II) salt or copper (I) salt.

In some example embodiments, the catalyst is selected from a group consisting of copper sulfate, copper bromide, and copper iodide.

In some example embodiments, the catalyst is a ruthenium catalyst.

In some example embodiments, the catalyst is selected from a group consisting of pentamethylcyclopentadienylbis(triphenylphosphine)ruthenium(II) chloride, pentamethylcyclopentadienyl(cyclooctadienyl)ruthenium(II) chloride, and pentamethylcyclopentadienyl(norbornadiene)ruthenium(II) chloride.

In some example embodiments, the base is selected from a group consisting of triethylamine, N,N-diisopropylethylamine, 4-(dimethylamino)pyridine, pyridine, quinolone, phenanthroline, and imidazole.

In some example embodiments, the reducing agent is selected from a group consisting of sodium ascorbate, tris(triazole)amine, and hydroquinones.

According to example embodiments, a method of isolating biomolecules of interest includes: (a) providing a functionalized substrate, where the functionalized substrate includes a substrate, one or more polymers, one or more azlactone groups attached to a first end of each of the one or more polymers, and one or more azide groups attached to a second end of each of the one or more polymers, the one or more azide groups thereby attaching each of the one or more polymers to the substrate; (b) providing an aqueous solution, where the aqueous solution contains the biomolecules of interest; and (c) contacting the functionalized substrate with the aqueous solution for a period of time, where during the period of time, the biomolecules of interest attach to the azlactone groups of the substrate.

In some example embodiments, the aqueous solution includes an additive, where the additive is selected from a group consisting of glycerol, oligoethylene glycol, polyethylene glycol, surfactants, polyvinylalcohol, sugars, organic solvents, and inorganic salts.

In some example embodiments, a pH level of the aqueous solution is in a range of from about 2 to about 10.

In some example embodiments, contacting the substrate with the aqueous solution is achieved by jet printing, pin printing, quill printing, biological laser printing, capillary-based fluidics, or immersion.

These and other features, aspects, and advantages of the present invention will become better understood when the following detailed description of certain exemplary embodiments is read with reference to the accompanying drawings in which like characters represent like parts throughout the drawings.

### BRIEF DESCRIPTION OF THE DRAWINGS

FIG. 1 illustrates a bifunctional polymer according to an example embodiment of the present invention.
FIG. 2 illustrates a coated substrate according to an example embodiment of the present invention.
FIG. 3 illustrates a microarray according to an example embodiment of the present invention.
FIG. 4 compares the fluorescence intensity of Green Fluorescent Protein (GFP) spots on NHS- and Azlactone-functionalized substrates (average of 10 spots).
FIG. 5A illustrates representative examples of GFP spot morphology obtained on NHS-functionalized substrates. FIG. 5B illustrates representative examples of GFP spot morphology obtained on azlactone-functionalized substrates.
FIG. 6 compares the fluorescence intensity of Immunoglobulin G (IgG) conjugated onto the surface of PEG-coated slides functionalized with azlactone groups and NHS groups.
FIG. 7 illustrates conversion of NHS-functionalized surfaces into azlactone-functionalized surfaces.
FIG. 8 illustrates conversion of aldehyde-functionalized surfaces into azlactone-functionalized surfaces.

### DETAILED DESCRIPTION

Example embodiments of the present invention provide a bifunctional polymer, such as PEG, functionalized at one end with an azlactone end group to conjugate biomolecules of interest, and functionalized at another end with an azide anchor group to attach the polymer to a substrate. Functionalizing a polymer with two groups that are chemically orthogonal, like azlactone and azide, allows azlactone functionality for conjugation of biomolecules to remain intact under conditions where the azide group is reactive for attaching the polymer to the substrate. Example embodiments of the present invention provide a coated substrate that incorporates such bifunctionalized polymers on the surface of a substrate, where the azide group attaches the polymer to the substrate, leaving the azlactone group free and active to conjugate biomolecules. Example embodiments of the present invention provide a microarray that incorporates such a coated substrate on the surface of the microarray, where the microarray contains a plurality of discrete spots, each spot containing the bifunctionalized polymers.

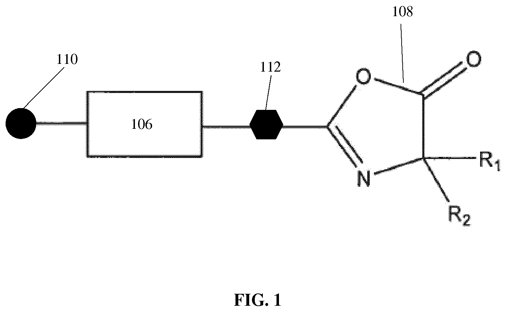

FIG. 1 shows a bifunctional polymer according to an example embodiment that includes an anchor group **110** selected from a group consisting of azide, carboxylic acid, thiol, amine, hydroxyl, hydrazine, silyl, phosphonate, alkyne, catechol, and lysine; a polymer block **106** that includes one or more first polymers, which can be PEG or polysaccharide; a linker group **112** selected from a group consisting of phenyl, vinyl, benzyl, and alkyl; and an azlactone end group **108** that includes R₁ and R₂, where R₁ and R₂ are each independently selected from a group consisting of hydrogen, alkyl, and aryl. In another example embodiment, the one or more first polymers can be copolymers with a second polymer, where the second polymer is selected from a group consisting of polylysine, polyoxazoline, polymethylmethacrylate, poly-N-isopropylacrylamide, polydopamine, polyalkane, and N-substituted glycine polymer.

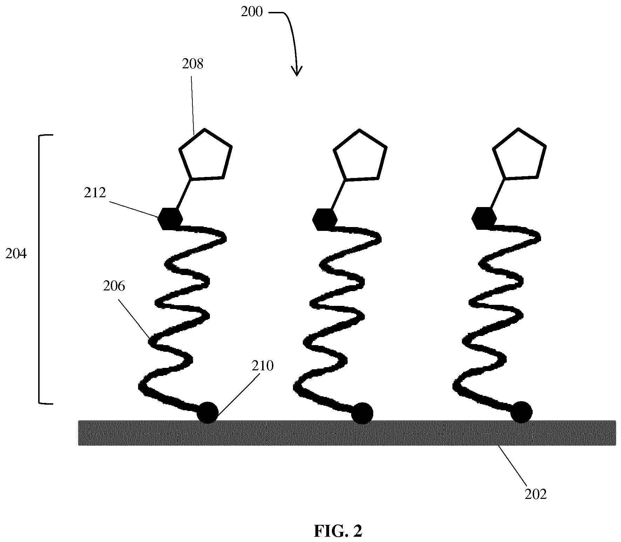

FIG. 2 shows a coated substrate **200** according to an example embodiment, the coated substrate **200** including substrate **202** and polymer layer **204** attached to substrate **202**, where polymer layer **204** includes one or more first polymers **206**, one or more azlactone functional groups **208** attached to a first end of each of the one or more first polymers **206** through a linker group **212**, and one or more azide groups **210** attached to a second end of each of the one or more first polymers **206**, where the one or more azide groups **210** attach polymer layer **204** to substrate **202.**

In example embodiments, substrate **202** can be glass, silica, plastic, carbon, metal, or metal oxide. As shown in FIG. 2, polymer layer **204** can be made of linear polymers, but in other example embodiments, polymer layer **204** can also be made of multiarm polymers, brush polymers, or nanoparticles. In some example embodiments, the one or more first polymers **206** can be PEG or polysaccharide, and in other example embodiments, the one or more first polymers **206** can be copolymers with a second polymer, where the second polymer can be polylysine, polyoxazoline, polymethylmethacrylate, poly-N-isopropylacrylamide, polydopamine, polyalkane, or N-substituted glycine polymer.

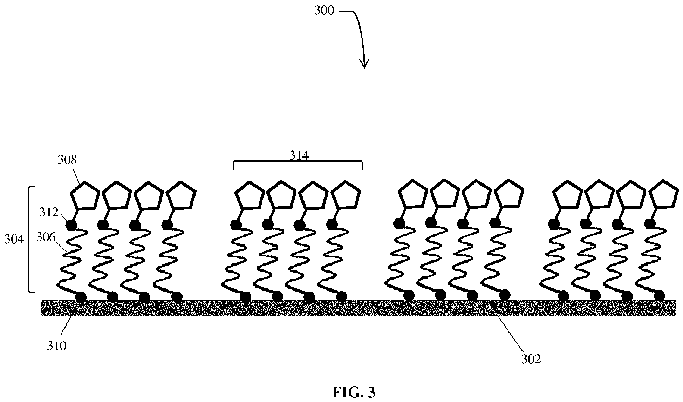

FIG. 3 shows a microarray **300** according to an example embodiment, the microarray **300** including a plurality of discrete regions **314**, where each of the discrete regions **314** includes a coated substrate that includes a substrate **302** and a polymer layer **304** attached to substrate **302**, where polymer layer **304** includes one or more polymers **306**, one or more azlactone functional groups **308** attached to a first end of each of the one or more polymers **306** through a linker group **312**, and one or more azide groups **310** attached to a second end of each of the one or more polymers **306**, where the one or more azide groups **310** attach polymer layer **304** to substrate **302.**

Example embodiments of the present invention also provide methods of preparing a bifunctional polymer, where a polymer, such as PEG, is functionalized with azlactone and azide groups.

According to example embodiments of the present invention, for example as described with respect to Examples 1 and 2 below, a method of preparing a bifunctional polymer includes: (a) providing a polymer, where the polymer contains one or more carboxylic acid groups or activated ester groups attached to a first end of the polymer and one or more azide groups attached to a second end of the polymer; (b) converting at least one carboxylic acid group or activated ester group into an aryl or vinyl halide group; and (c) attaching vinyldialkyl azlactone to the aryl or vinyl halide group. The polymer can be PEG. The vinyldialkyl azlactone can be attached to the aryl or vinyl halide group through a coupling reaction using a palladium catalyst, one or more solvents, and a base. The palladium catalyst can be selected from a group consisting of palladium (II) quinoline-8-carboxylate, palladium (II) chloride, palladium (II) bromide, palladium (II) acetate, palladium (II) acetoacetate, bis(dibenzylideneacetone)palladium(0), tris(dibenzylideneacetone)dipalladium(0), palladium (II) trifluoroacetate, allylpalladium (II) chloride dimer, bis(triphenylphosphine)palladium(II) dichloride, dichlorobis(tricyclohexylphosphine)palladium(II), and [1,1'-bis(di-tert-butylphosphino)ferrocene]dichloropalladium(II). The one or more solvents can be selected from a group consisting of dimethylformamide, dimethylsulfoxide, toluene, tetrahydrofuran, dioxane, dichloromethane, acetonitrile, and alcohol. The base can be selected from a group consisting of potassium carbonate, sodium carbonate, triethylamine, N,N-diisopropylethylamine, 4-(dimethylamino)pyridine, potassium tert-butoxide, and sodium tert-butoxide.

Example embodiments of the present invention also provide methods for functionalizing a surface with azlactone functionalized polymers, such as PEG. According to an example embodiment of the present invention, for example as described with respect to Example 3 below, there is provided a "top down" method of attaching to a substrate a bifunctional polymer that includes a polymer, one or more azlactone functional groups attached to a first end of the polymer, and one or more azide groups attached to a second end of the polymer, the method including: (a) providing a substrate containing alkyne functional groups; (b) providing a mixture that includes the bifunctional polymer, one or more solvents, a catalyst, a base, and a reducing agent; and (c) contacting the substrate with the mixture.

In the top down approach, the azlactone group and azido group are introduced to the ends of the polymer chain first, and then the polymer is attached to the surface containing alkyne groups via "click" reaction. This top down approach permits better control over the degree of functionalization, ensuring that each polymer chain has azlactone functionality. The substrate can be metal, metal oxide, silica, glass, carbon, or plastic. Methods of introducing alkyne functionality onto such surfaces are known. For example, Achatz et al., "Colloidal silica nanoparticles for use in click chemistry-based conjugations and fluorescent affinity assays," Sensors and Actuators B: Chemistry, 150(1):211-19 (2010), discloses a method of decorating silica nanoparticles with O-(propargyl)-N-(triethoxysilylpropyl) carbamate. The polymer can be PEG or polysaccharides. The one or more solvents can be selected from a group consisting of dimethylformamide, dimethylsulfoxide, toluene, tetrahydrofuran, dioxane, acetonitrile, and water. The catalyst can be either a copper (II) salt or copper (I) salt (including copper sulfate, copper bromide, and copper iodide) or a ruthenium catalyst (including pentamethylcyclopentadienylbis(triphenylphosphine)ruthenium(II) chloride, pentamethylcyclopentadienyl(cyclooctadienyl)ruthenium(II) chloride, and pentamethylcyclopentadienyl(norbornadiene)ruthenium(II) chloride). The base can be selected from a group consisting of triethylamine, N,N-diisopropylethylamine, 4-(dimethylamino)pyridine, pyridine, quinolone, phenanthroline, and imidazole. The reducing agent can be selected from a group consisting of sodium ascorbate, tris(triazole)amine, and hydroquinones. Contacting the substrate with the mixture can be done at room temperature or elevated temperatures, such as from about 23 to 150° C, and the amount of reaction time permitted for contacting the substrate with the mixture correlates with the resulting density of PEG coating.

According to an alternative example embodiment of the present invention, a "bottom up" method of functionalizing a surface with azlactone includes providing a polymer, where the polymer contains one or more carboxylic acid groups attached to a first end of the polymer and one or more attachment groups attached to a second end of the polymer; attaching the polymer to the surface through the attachment group; and then converting the carboxylic acid group into azlactone. The polymer can be PEG. This bottom up approach permits the use of more alternative chemistries for attachment of the polymer to the surface, since the surface attachment is no longer required to be orthogonal to azlactone chemistry. In the bottom up approach, the attachment group of the polymer can be azide, phosphonic acid, carboxylic acid, silane, thiol, amine, hydrazine, alkyne, or catechol.

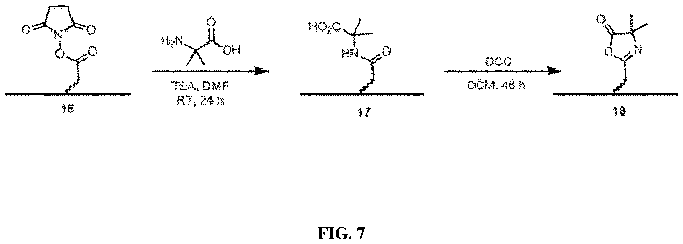

According to an example embodiment of the present invention, as shown in FIG. 7, an NHS-functionalized surface (**16**) is converted into an azlactone-functionalized surface (**18**) through solid phase synthesis. The NHS-functionalized surface is reacted with 2-methylalanine in the presence of a base (for example, trimethylamine (TEA)) and a solvent (for example, dimethylformamide (DMF)), resulting in intermediate structure (**17**). Intermediate structure (**17**) undergoes a cyclization reaction in the presence of a coupling reagent (for example, a carbodiimide, such as N,N'-dicyclohexylcarbodiimide (DCC)) and a solvent (for example, dichloromethane (DCM)), resulting in azlactone-functionalized surface (**18**).

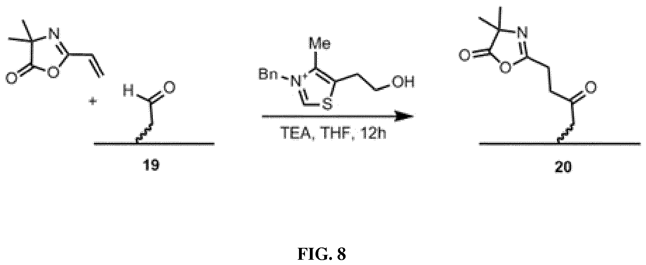

According to another example embodiment of the present invention, as shown in FIG. 8, an aldehyde-functionalized surface (**19**) is converted into an azlactone-functionalized surface (**20**) through solid phase synthesis. The aldehyde-functionalized surface (**19**) is reacted with vinyldialkyl azlactone in the presence of a catalyst (for example, 3-Benzyl-5-(2-hydroxyethyl)-4-methylthiazolium chloride), a base (for example, TEA), and a solvent (for example, tetrahydrofuran (THF)), resulting in the azlactone-functionalized surface (**20**).

A substrate with azlactone functional groups provides certain advantages, including a wider range of reaction conditions compatible with the substrate. Due to hydrolytic stability of azlactone groups, the substrate will tolerate a variety of buffers, pH levels, and other environmental conditions (including temperature and humidity), thus making it easier for a user to optimize biomolecule attachment chemistry and achieve improved signal intensity and uniformity.

For example, when using the spotting method to produce protein arrays on solid substrates, a drop of protein solution is dispensed onto the substrate and allowed to dry. The drying process influences the signal intensity and uniformity, where a slower drying process should improve conjugation efficiency (since there is more time for the reaction to happen) as well as minimize the coffee ring effect, where a circular outer perimeter contains higher protein concentration than the center. However, with groups such as NHS, activated esters, epoxides, and aldehydes, water hydrolysis competes with conjugation reaction, and therefore faster drying is preferred at the expense of better intensity and uniformity. The robustness of azlactone groups in aqueous solution allows for a slower drying process to achieve both optimal signal intensity and uniformity.

As another example, continuous-flow microprinting is another method of producing protein arrays. Continuous-flow microprinting provides longer contact of a functional substrate with a protein solution without drying and can be utilized to its full potential if a substrate has hydrolytically stable functional groups on the surface.

Example embodiments of the present invention also provide methods of using an azlactone functionalized substrate to conjugate biomolecules of interest.

According to an example embodiment of the present invention, a method of isolating biomolecules of interest includes: (a) providing a functionalized substrate that includes a substrate, one or more polymers, one or more azlactone groups attached to a first end of each of the one or more polymers, and one or more azide groups attached to a second end of each of the one or more polymers, where the one or more azide groups attach each of the one or more polymers to the substrate; (b) providing an aqueous solution that contains the biomolecules of interest; and (c) contacting the functionalized substrate with the aqueous solution for a period of time, where during the period of time, the biomolecules of interest attach to the azlactone groups. The aqueous solution can include an additive (such as glycerol, oligoethylene glycol, polyethylene glycol, surfactants, polyvinylalcohol, sugars, organic solvents, and inorganic salts), and can have a pH level in a range of from about 2 to about 10. Contacting the functionalized substrate with the aqueous solution can be achieved by jet printing, pin printing, quill printing, biological laser printing, capillary-based fluidics, or immersion. The period of time can be from several seconds to several hours. Additionally, biomolecules can be expressed and captured on the substrate in-situ using in vitro transcription and translation technology.

The above description is intended to be illustrative, and not restrictive. Those skilled in the art can appreciate from the foregoing description that the present invention may be implemented in a variety of forms, and that the various embodiments can be implemented alone or in combination. Therefore, while the embodiments of the present invention have been described in connection with particular examples thereof, the true scope of the embodiments and/or methods of the present invention should not be so limited since other modifications will become apparent to the skilled practitioner upon a study of the drawings, specification, and the following examples and claims.

The following are examples which illustrate specific methods without the intention to be limiting in any manner. The examples may be modified within the scope of the description as would be understood from the prevailing knowledge.

### EXAMPLES

### Example 1 - Synthesis of Azido-PEG-Azlactone, Converting Carboxylic Acid Group into an Aryl Halide Group

A mixture of N₃-PEG₁ₖ-CO₂H (200 mg, 0.20 mmol, 1.0 equiv), 1-ethyl-3-[3-dimethylaminopropyl] carbodiimide hydrochloride (EDC) (37 mg, 0.24 mmol, 1.2 equiv), and iodoaniline (52 mg, 0.24 mmol, 1.2 equiv) in dry dichloromethane (DCM) was stirred under argon at room temperature for 12 hours. The reaction was quenched with water, and the organic layer was separated. The aqueous layer was extracted with DCM three times, and combined organic portions were dried over magnesium sulfate (MgSO₄). The solution was concentrated under reduced pressure to a crude oil, which was purified by silica gel column chromatography (0-10% methanol/DCM) to yield a soft white solid (225 mg, 82%).

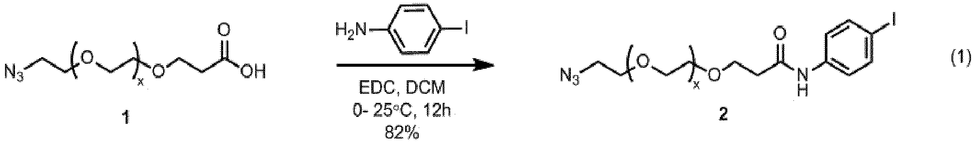

The structure of the resulting product was determined using proton nuclear magnetic resonance (NMR) spectroscopy, with the resulting NMR spectrum: ¹H NMR (600 MHz, CDCl3) δ = 8.82 (bs, 1H), 7.58 (d, J = 9.1 Hz, 2H), 7.39 (d, J = 9.1 Hz, 2H), 3.81 (t, J = 5.9 Hz, 2H), 3.70-3.59 (m, 96H), 3.38 (t, J = 5.4 Hz, 2H), 2.63 (t, J = 5.4 Hz, 2H) ppm; ¹³C NMR (125 MHz, CDCl3) δ = 186.8, 137.8, 136.2, 123.2, 122.2, 82.9, 70.7, 38.1 ppm. The retention factor (R_{f}) of the product in thin layer chromatography (TLC) is as follows: TLC R_{f} = 0.5 (10% MeOH/DCM).

### Example 2 - Synthesis of Azido-PEG-Azlactone, Attaching Vinyldialkyl Azlactone to the Aryl Halide Group

Palladium catalyst "Quin₂Pd" was synthesized according to a procedure such as that described in Cui et al., "Pd(quinoline-8-carboxylate)2 as a Low-Priced, Phosphine-Free Catalyst for Heck and Suzuki Reactions," Journal of Organic Chemistry, 72:9342 (2007).

A 10 mL flame-dried Schlenk flask was charged with N₃-PEG₁ₖ-iodoanilide (200 mg, 0.152 mmol, 1.0 equiv) and Quin-Pd (3.2 mg, 0.0072 mmol, 0.05 equiv) and purged with argon. Dry dimethylformamide (DMF) (0.2 M, 0.8 mL), triethylamine (100 µL, 0.076 mmol, 5.0 equiv, dry and freshly distilled over CaH₂), and vinyl azlactone (58 µL, 0.456 mmol, 3.0 equiv) were added and the system was sealed. The reaction mixture was heated at 130 °C for 3 hours, turning a dark brown. It was then cooled to room temperature and concentrated under reduced pressure. The crude residue was dissolved in DCM and dry loaded onto Celite, which was applied to a silica column. Purification by column chromatography (0-20% methanol/DCM) yielded 156 mg.

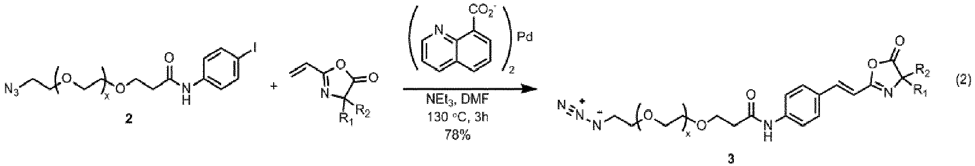

The structure of the resulting product was determined using proton NMR spectroscopy, with the resulting NMR spectrum: ¹H NMR (600 MHz) δ = 9.10 (bs, 1H), 7.67 (d, J= 8.0 Hz, 2H), 7.43 (d, J = 8.0 Hz, 2H), 7.41 (d, J = 15.4 Hz, 1 H), 6.47 (d, J = 16.5 Hz, 1 H), 3.81 (t, J = 6.0 Hz, 2H), 3.64-3.57 (m, -107 H), 2.67 (t, J = 5.2 Hz, 2H), 1.46 (s, 6H) ppm; ¹³C NMR (125 MHz) δ = 181.0, 170.5, 159.6, 142.3, 140.7, 128.6, 120.1, 112.0, 70.5, 67.2, 46.4, 38.0, 24.9 ppm. The retention factor of the product in thin layer chromatography is as follows: TLC Rf = 0.4 (10% MeOH/DCM).

### Example 3 - Attaching Azido-PEG-Azlactone onto Alkyne-Functionalized Surface via "Click" Reaction

To a solution (3 mg/mL) of azide-PEG-azlactone in 5 mL dimethyl sulfoxide (DMSO) and 5 mL deionized (DI) water was added 0.52 mL of 1.0 mM stock click chemistry solution (1.5 mg CuSO₄·5H₂O, 6 mg sodium ascorbate, 4 µL triethylamine, 3 mL DMSO, and 3 mL DI water). The slides functionalized with alkyne groups were immersed in this solution with gentle shaking for 12 hours at room temperature. The PEGylated slides were rinsed with DI water and spun dry.

Alternatively, PEG-azlactone was introduced onto a surface by pre-functionalizing the surface with azide groups, then coupling the surface with alkyne-PEG-azlactone via "click" reaction.

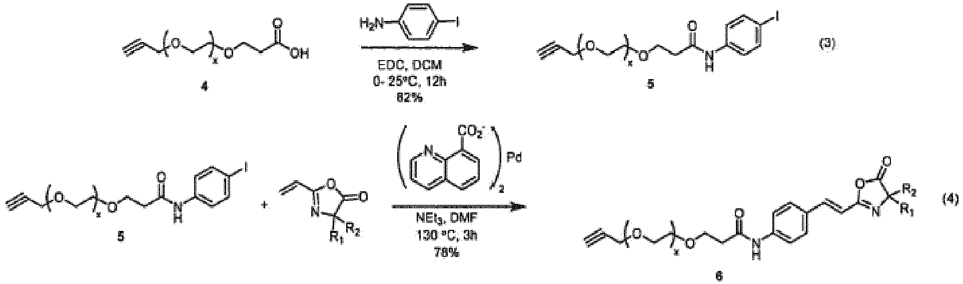

### Example 4 - Synthesis of Polysaccharides Modified with Azlactone and Azide Functional Groups

As illustrated below, polysaccharides were oxidized to produce carboxylic acid groups suitable for further functionalization with azlactone. Some of the primary alcohols on polysaccharide chain (structure **7**) were converted into carboxylic acid groups either using oxygen over platinum or using TEMPO ((2,2,6,6-Tetramethylpiperidin-1-yl)oxyl or (2,2,6,6-tetramethylpiperidin-1-yl)oxidanyl) as a catalyst and sodium hypochlorite (NaOCl) in basic pH, resulting in structure (**8**) (see Cumpstey, I., ISRN Organic Chemistry, Article ID 417672 (2013)). The carboxylic acid groups were then converted into aryl or vinyl halide (bromide or iodide) functionality, resulting in structure **9**, followed by attachment of vinyldialkyl azlactone via palladium-catalyzed coupling to produce the desired azlactone-functionalized material (structure **10**).

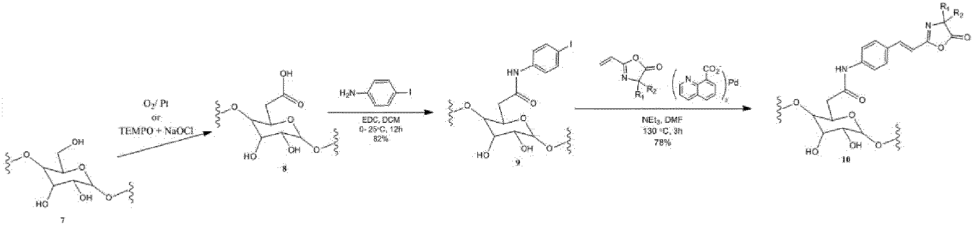

Alternatively, as illustrated below, periodate was used to achieve cleavage of 1,2 diols of the polysaccharide chain (structure **7**) and introduce carboxylic acid functionality into the polymer chain, resulting in structure **11.** Structure **11** was then reacted with iodoaniline, resulting in structure **12**, which was then reacted with vinyldialkyl azlactone to produce the desired azlactone-functionalized material (structure **13**).

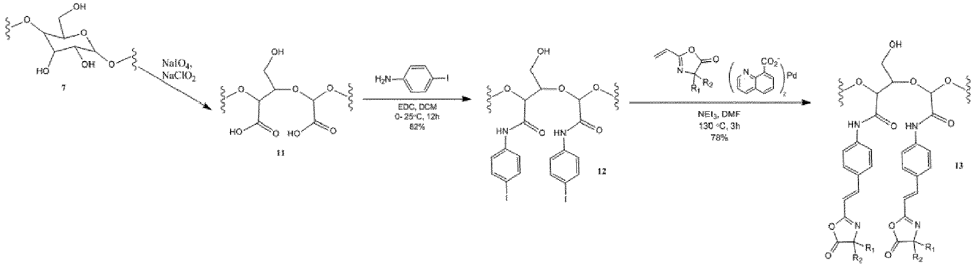

As illustrated below, azide functionality was introduced into the polysaccharide chain (structure **7**) by converting some of the primary alcohols of the polysaccharide chain into bromides (for example, using N-bromosuccinimide (NBS) and triphenylphosphine (PPh)) or tosyl groups to give structure **14** (by reacting it with tosyl chloride (TsCl)) followed by reaction with sodium azide (NaN₃) to produce structure **15** (see Cumpstey). This method can be combined with either of the two prior methods of synthesizing azlactone functionalized polysaccharides to yield a polysaccharide chain containing both azlactone and azide groups. Since only a few of the primary alcohols on a polysaccharide chain are modified into azlactones, the other alcohols are available for conversion into azides.

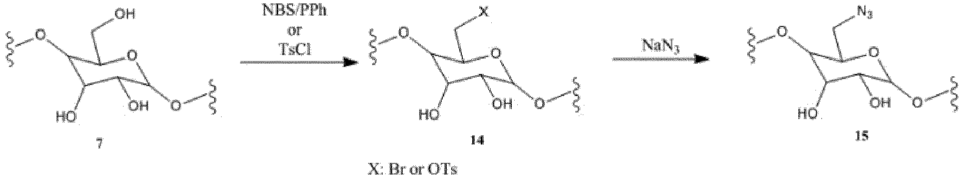

### Example 5 - Attaching Protein to Substrate Coated with Azido-PEG-Azlactone via Pin Printing

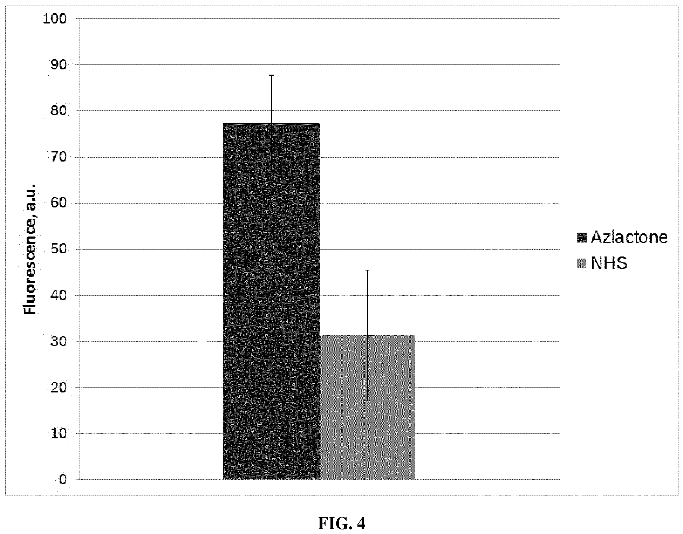

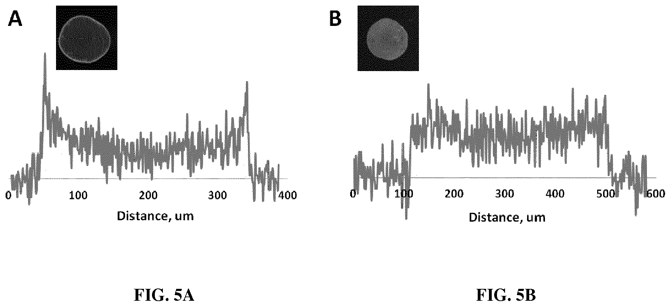

200 mg/mL solution of Green Fluorescent Protein (GFP) containing 0.1% of polyvinyl alcohol in phosphate buffered saline (PBS) was spotted onto the substrate using a pin printing method at room temperature and relative humidity of 55%. The spots were allowed to dry for 12 hours in a desiccator, and then the slides were rinsed with PBS-Tween and PBS to remove unbound protein. Relative amounts of GFP covalently attached to the substrate were determined by measuring the fluorescent intensity of the protein on the surface. The fluorescence intensity of azlactone-functionalized slides was 2.5 times higher compared to NHS ester-functionalized slides (see FIG. 4). Morphology of the slides was compared by looking at the fluorescence intensity across the spots. Protein spots on azlactone-functionalized surfaces were uniform (FIG. 5B), while spots on NHS-functionalized surfaces displayed characteristic coffee ring morphology (see FIG. 5A).

### Example 6 - Protein Conjugation via Immersion

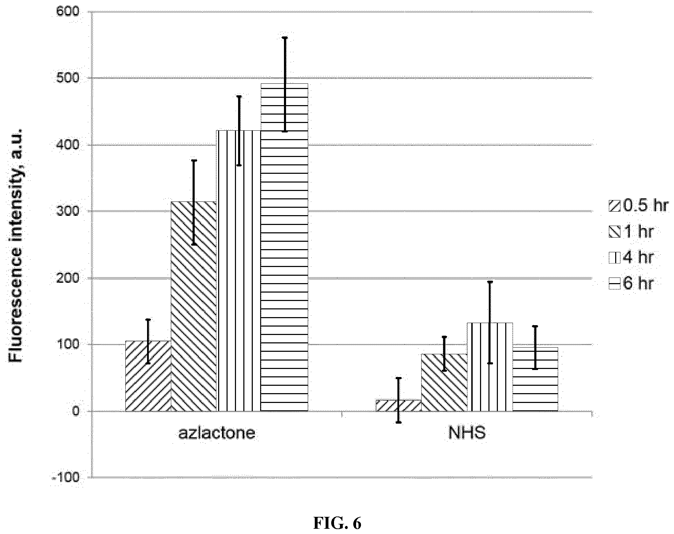

Glass substrates functionalized with azide-PEG-azlactone and azide-PEG-NHS containing the same density of functional groups on the surface were exposed to a solution of Immunoglobulin G (IgG) in phosphate buffered saline (PBS) at pH 7.4 for up to 6 hours. After removing the solution and rinsing the slides with PBS-Tween and PBS, the relative amounts of IgG covalently attached to the substrates were determined by measuring the fluorescent intensity of the protein on the surface. After 6 hours of incubation, fluorescence intensity of the azlactone-functionalized slides was 5 times higher compared to that of the NHS ester-functionalized slides (see FIG. 6).

## Claims

1. A bifunctional polymer comprising:
(a) an anchor group selected from a group consisting of azide, carboxylic acid, thiol, amine, hydroxyl, hydrazine, silyl, phosphonate, alkyne, catechol, and lysine;
(b) a polymer block that includes one or more first polymers, the one or more first polymers including polyethylene glycol or polysaccharide;
(c) a linker group selected from a group consisting of phenyl, vinyl, benzyl, and alkyl; and
(d) an azlactone end group containing R₁ and R₂, wherein R₁ and R₂ are each independently selected from a group consisting of hydrogen, alkyl, and aryl.

2. The bifunctional polymer of claim 1, wherein the one or more first polymers is a copolymer with a second polymer that is selected from a group consisting of polylysine, polyoxazoline, polymethylmethacrylate, poly-N-isopropylacrylamide, polydopamine, polyalkane, and N-substituted glycine polymer.

3. A coated substrate comprising:
(a) a substrate; and
(b) a polymer layer that includes one or more first polymers, one or more azlactone functional groups attached to a first end of each of the one or more first polymers, and one or more azide groups attached to a second end of each of the one or more first polymers, wherein the one or more azide groups attach the polymer layer to the substrate.

4. The coated substrate of claim 3, wherein the substrate is selected from a group consisting of glass, silica, plastic, carbon, metal, and metal oxide.

5. The coated substrate of claim 3, wherein the polymer layer comprises linear polymer, multiarm polymer, brush polymer, or nanoparticles.

6. The coated substrate of claim 3, wherein the one or more first polymers includes polyethylene glycol or polysaccharide.

7. The coated substrate of claim 6, wherein the one or more first polymers is a copolymer with a second polymer that is selected from a group consisting of polylysine, polyoxazoline, polymethylmethacrylate, poly-N-isopropylacrylamide, polydopamine, polyalkane, and N-substituted glycine polymer.

8. A microarray comprising:
a plurality of discrete regions that each includes a coated substrate, the coated substrate comprising:
(a) a substrate; and
(b) a polymer layer that includes one or more polymers, one or more azlactone functional groups attached to a first end of each of the one or more polymers, and one or more azide groups attached to a second end of each of the one or more polymers, wherein the one or more azide groups attaches the polymer layer to the substrate.

9. A method of preparing a bifunctional polymer, the method comprising:
(a) providing a polymer, wherein the polymer contains one or more carboxylic acid groups or activated ester groups attached to a first end of the polymer and one or more azide groups attached to a second end of the polymer;
(b) converting at least one carboxylic acid group or activated ester group into an aryl or vinyl halide group; and
(c) attaching vinyldialkyl azlactone to the aryl or vinyl halide group.

10. The method of claim 9, wherein the polymer is polyethylene glycol.

11. The method of claim 9, wherein the vinyldialkyl azlactone is attached through a coupling reaction using a palladium catalyst, one or more solvents, and a base.

12. The method of claim 11, wherein the palladium catalyst is selected from a group consisting of palladium (II) quinoline-8-carboxylate, palladium (II) chloride, palladium (II) bromide, palladium (II) acetate, palladium (II) acetoacetate, bis(dibenzylideneacetone)palladium(0), tris(dibenzylideneacetone)dipalladium(0), palladium (II) trifluoroacetate, allylpalladium (II) chloride dimer, bis(triphenylphosphine)palladium(II) dichloride, dichlorobis(tricyclohexylphosphine)palladium(II), and [1,1-bis(di-tert-butylphosphino)ferrocene]dichloropalladium(II).

13. The method of claim 11, wherein the one or more solvents is selected from a group consisting of dimethylformamide, dimethylsulfoxide, toluene, tetrahydrofuran, dioxane, dichloromethane, acetonitrile, and alcohol.

14. The method of claim 11, wherein the base is selected from a group consisting of potassium carbonate, sodium carbonate, triethylamine, N,N-diisopropylethylamine, 4-(dimethylamino)pyridine, potassium tert-butoxide, and sodium tert-butoxide.

15. A method of attaching a bifunctional polymer to a substrate, the method comprising:
(a) providing a substrate containing alkyne functional groups;
(b) providing a mixture comprising the bifunctional polymer, one or more solvents, a catalyst, a base, and a reducing agent; and
(c) contacting the substrate with the mixture;
wherein the bifunctional polymer comprises a polymer, one or more azlactone functional groups attached to a first end of the polymer, and one or more azide groups attached to a second end of the polymer.

16. The method of claim 15, wherein the polymer is polyethylene glycol.

17. The method of claim 15, wherein the one or more solvents is selected from a group consisting of dimethylformamide, dimethylsulfoxide, toluene, tetrahydrofuran, dioxane, acetonitrile, and water.

18. The method of claim 15, wherein the catalyst comprises a copper (II) salt or copper (I) salt.

19. The method of claim 18, wherein the catalyst is selected from a group consisting of copper sulfate, copper bromide, and copper iodide.

20. The method of claim 15, wherein the catalyst comprises a ruthenium catalyst.

21. The method of claim 20, wherein the catalyst is selected from a group consisting of pentamethylcyclopentadienylbis(triphenylphosphine)ruthenium(II) chloride, pentamethylcyclopentadienyl(cyclooctadienyl)ruthenium(II) chloride, and pentamethylcyclopentadienyl(norbornadiene)ruthenium(II) chloride.

22. The method of claim 15, wherein the base is selected from a group consisting of triethylamine, N,N-diisopropylethylamine, 4-(dimethylamino)pyridine, pyridine, quinolone, phenanthroline, and imidazole.

23. The method of claim 15, wherein the reducing agent is selected from a group consisting of sodium ascorbate, tris(triazole)amine, and hydroquinones.

24. A method of isolating biomolecules of interest, the method comprising:
(a) providing a functionalized substrate, the functionalized substrate comprising a substrate, one or more polymers, one or more azlactone groups attached to a first end of each of the one or more polymers, and one or more azide groups attached to a second end of each of the one or more polymers, wherein the one or more azide groups attaches each of the one or more polymers to the substrate;
(b) providing an aqueous solution, wherein the aqueous solution contains the biomolecules of interest; and
(c) contacting the functionalized substrate with the aqueous solution for a period of time, wherein during the period of time, the biomolecules of interest attach to the azlactone groups.

25. The method of claim 24, wherein the aqueous solution further comprises an additive, wherein the additive is selected from a group consisting of glycerol, oligoethylene glycol, polyethylene glycol, surfactants, polyvinylalcohol, sugars, organic solvents, and inorganic salts.

26. The method of claim 24, wherein a pH level of the aqueous solution is in a range of from about 2 to about 10.

27. The method of claim 24, wherein the contacting of the functionalized substrate with the aqueous solution is achieved by jet printing, pin printing, quill printing, biological laser printing, capillary-based fluidics, or immersion.

## Patentansprüche

1. Bifunktionelles Polymer, umfassend:
(a) eine Ankergruppe ausgewählt aus einer Gruppe bestehend aus Azid, Carbonsäure, Thiol, Amin, Hydroxy, Hydrazin, Silyl, Phosphonat, Alkin, Catechol und Lysin;
(b) einen Polymerblock, der ein oder mehrere erste Polymere umfasst, wobei das eine oder die mehreren ersten Polymere Polyethylenglycol oder Polysaccharid umfassen;
(c) eine Linkergruppe ausgewählt aus einer Gruppe bestehend aus Phenyl, Vinyl, Benzyl und Alkyl; und
(d) eine Azlacton-Endgruppe, die R₁ und R₂ enthält, wobei R₁ und R₂ jeweils unabhängig ausgewählt sind aus einer Gruppe bestehend aus Wasserstoff, Alkyl und Aryl.

2. Bifunktionelles Polymer gemäß Anspruch 1, wobei das eine oder die mehreren ersten Polymere ein Copolymer mit einem zweiten Polymer ist, das ausgewählt ist aus einer Gruppe bestehend aus Polylysin, Polyoxazolin, Polymethylmethacrylat, Poly-N-isopropylacrylamid, Polydopamin, Polyalkan und N-substituiertem Glycinpolymer.

3. Beschichtetes Substrat, umfassend:
(a) ein Substrat; und
(b) eine Polymerschicht, die ein oder mehrere erste Polymere, eine oder mehrere Azlacton-funktionelle Gruppen, die an einem ersten Ende von jedem von dem einen oder den mehreren ersten Polymeren befestigt sind, und eine oder mehrere Azidgruppen, die an einem zweiten Ende von jedem von dem einen oder den mehreren ersten Polymeren befestigt sind, wobei die eine oder mehreren Azidgruppen die Polymerschicht an dem Substrat befestigen.

4. Beschichtetes Substrat gemäß Anspruch 3, wobei das Substrat ausgewählt ist aus einer Gruppe bestehend aus Glas, Siliciumdioxid, Kunststoff, Kohlenstoff, Metall und Metalloxid.

5. Beschichtetes Substrat Anspruch 3, wobei die Polymerschicht lineares Polymer, mehrarmiges Polymer, Bürstenpolymer oder Nanopartikel umfasst.

6. Beschichtetes Substrat Anspruch 3, wobei das eine oder die mehreren ersten Polymere Polyethylenglycol oder Polysaccharid umfassen.

7. Beschichtetes Substrat Anspruch 6, wobei das eine oder die mehreren ersten Polymere ein Copolymer mit einem zweiten Polymer ist, das ausgewählt ist aus einer Gruppe bestehend aus Polylysin, Polyoxazolin, Polymethylmethacrylat, Poly-N-isopropylacrylamid, Polydopamin, Polyalkan und N-substituiertem Glycinpolymer.

8. Mikroarray, umfassend:
eine Vielzahl von diskreten Bereichen, die jeweils ein beschichtetes Substrat umfassen, wobei das beschichtete Substrat umfasst:
(a) ein Substrat; und
(b) eine Polymerschicht, die ein oder mehrere erste Polymere, eine oder mehrere Azlacton-funktionelle Gruppen, die an einem ersten Ende von jedem von dem einen oder den mehreren ersten Polymeren befestigt sind, und eine oder mehrere Azidgruppen, die an einem zweiten Ende von jedem von dem einen oder den mehreren ersten Polymeren befestigt sind, wobei die eine oder mehreren Azidgruppen die Polymerschicht an dem Substrat befestigen.

9. Verfahren zur Herstellung eines bifunktionellen Polymers, wobei das Verfahren umfasst:
(a) Bereitstellen eines Polymers, wobei das Polymer eine oder mehrere Carbonsäuregruppen oder aktivierte Estergruppen, die an ein erstes Ende des Polymers gebunden sind, und eine oder mehrere Azidgruppen, die an ein zweites Ende des Polymers gebunden sind, aufweist;
(b) Umwandeln wenigstens einer Carbonsäuregruppe oder aktivierten Estergruppe in eine Aryl- oder Vinylhalogenidgruppe; und
(c) Befestigen von Vinyldialkylazlacton an der Aryl- oder Vinylhalogenidgruppe.

10. Verfahren gemäß Anspruch 9, wobei das Polymer Polyethylenglycol ist.

11. Verfahren gemäß Anspruch 9, wobei das Vinyldialkylazlacton durch eine Kopplungsreaktion unter Verwendung eines Palladiumkatalysators, eines oder mehrerer Lösungsmittel und einer Base befestigt wird.

12. Verfahren gemäß Anspruch 11, wobei der Palladiumkatalysator ausgewählt ist aus einer Gruppe bestehend aus Palladium(II)-chinolin-8-carboxylat, Palladium(II)-chlorid, Palladium(II)-bromid, Palladium(II)-acetat, Palladium(II)-acetoacetat, Bis(dibenzylidenaceton)palladium(0), Tris(dibenzylidenaceton)dipalladium(0), Palladium(II)-trifluoracetat, Allylpalladium(II)-chlorid-Dimer, Bis(triphenylphosphin)palladium(II)-dichlorid, Dichlorbis(tricyclohexylphosphin)palladium(II) und [1,1'-Bis(di-tert-butylphosphino)ferrocen]dichlorpalladium(II).

13. Verfahren gemäß Anspruch 11, wobei das eine oder die mehreren Lösungsmittel ausgewählt sind aus einer Gruppe bestehend aus Dimethylformamid, Dimethylsulfoxid, Toluol, Tetrahydrofuran, Dioxan, Dichlormethan, Acetonitril und Alkohol.

14. Verfahren gemäß Anspruch 11, wobei die Base ausgewählt ist aus einer Gruppe bestehend aus Kaliumcarbonat, Natriumcarbonat, Triethylamin, N,N-Diisopropylethylamin, 4-(Dimethylamino)pyridin, Kalium-tert-butoxid und Natrium-tert-butoxid.

15. Verfahren zum Befestigen eines bifunktionellen Polymers an ein Substrat, wobei das Verfahren umfasst:
(a) Bereitstellen eines Substrats, das alkinfunktionelle Gruppen aufweist;
(b) Bereitstellen eines Gemischs, das das bifunktionelle Polymer, ein oder mehrere Lösungsmittel, einen Katalysator, eine Base und ein Reduktionsmittel umfasst; und
(c) Inkontaktbringen des Substrats mit dem Gemisch;
wobei das bifunktionelle Polymer ein Polymer, eine oder mehrere Azlacton-funktionelle Gruppen, die an einem ersten Ende des Polymers befestigt sind, und eine oder mehrere Azidgruppen, die an einem zweiten Ende des Polymers befestigt sind, umfasst.

16. Verfahren gemäß Anspruch 15, wobei das Polymer Polyethylenglycol ist.

17. Verfahren gemäß Anspruch 15, wobei das eine oder die mehreren Lösungsmittel ausgewählt sind aus einer Gruppe bestehend aus Dimethylformamid, Dimethylsulfoxid, Toluol, Tetrahydrofuran, Dioxan, Acetonitril und Wasser.

18. Verfahren gemäß Anspruch 15, wobei der Katalysator ein Kupfer(II)-Salz oder Kupfer(I)-Salz umfasst.

19. Verfahren gemäß Anspruch 18, wobei der Katalysator ausgewählt ist aus einer Gruppe bestehend aus Kupfersulfat, Kupferbromid und Kupferiodid.

20. Verfahren gemäß Anspruch 15, wobei der Katalysator einen Rutheniumkatalysator umfasst.

21. Verfahren gemäß Anspruch 20, wobei der Katalysator ausgewählt ist aus einer Gruppe bestehend aus Pentamethylcyclopentadienylbis(triphenylphosphin)-ruthenium(II)-Chlorid, Pentamethylcyclopentadienyl(cyclooctadienyl)-ruthenium(II)-Chlorid und Pentamethylcyclopentadienyl(norbornadien)-ruthenium(II)-Chlorid.

22. Verfahren gemäß Anspruch 15, wobei die Base ausgewählt ist aus einer Gruppe bestehend aus Triethylamin, N,N-Diisopropylethylamin, 4-(Dimethylamino)pyridin, Pyridin, Chinolon, Phenanthrolin und Imidazol.

23. Verfahren gemäß Anspruch 15, wobei das Reduktionsmittel ausgewählt ist aus einer Gruppe bestehend aus Natriumascorbat, Tris(triazol)amin und Hydrochinonen.

24. Verfahren zum Isolieren von Biomolekülen von Interesse, wobei das Verfahren umfasst:
(a) Bereitstellen eines funktionalisierten Substrats, wobei das funktionalisierte Substrat ein Substrat, ein oder mehrere Polymere, eine oder mehrere Azlactongruppen, die an einem ersten Ende von jedem des einen oder der mehreren Polymere befestigt sind, und eine oder mehrere Azidgruppen, die an einem zweiten Ende von jedem des einen oder der mehreren Polymere befestigt sind, umfasst, wobei die eine oder mehreren Azidgruppen jedes von dem einen oder den mehreren Polymeren an dem Substrat befestigen;
(b) Bereitstellen einer wässrigen Lösung, wobei die wässrige Lösung die Biomoleküle von Interesse enthält; und
(c) Inkontaktbringen des funktionalisierten Substrats mit der wässrigen Lösung für einen Zeitraum, wobei während des Zeitraums die Biomoleküle von Interesse an die Azlactongruppen binden.

25. Verfahren gemäß Anspruch 24, wobei die wässrige Lösung ferner einen Zusatzstoff umfasst, wobei der Zusatzstoff ausgewählt ist aus einer Gruppe bestehend aus Glycerol, Oligoethylenglycol, Polyethylenglycol, grenzflächenaktiven Mitteln, Polyvinylalkohol, Zuckern, organischen Lösungsmitteln und anorganischen Salzen.

26. Verfahren gemäß Anspruch 24, wobei das pH-Niveau der wässrigen Lösung in einem Bereich von etwa 2 bis etwa 10 liegt.

27. Verfahren gemäß Anspruch 24, wobei das Inkontaktbringen des funktionalisierten Substrats mit der wässrigen Lösung durch Strahldrucken, Stiftdrucken, Federdrucken, biologisches Laserdrucken, Fluidik auf Kapillarbasis oder Eintauchen durchgeführt wird.

## Revendications

1. Polymère bifonctionnel comprenant :
(a) un groupe d'ancrage choisi dans un groupe constitué d'azide, acide carboxylique, thiol, amine, hydroxyle, hydrazine, silyle, phosphonate, alcyne, catéchol et lysine ;
(b) un bloc de polymère qui comprend un ou plusieurs premiers polymères, les un ou plusieurs premiers polymères comprenant le polyéthylène glycol ou un polysaccharide ;
(c) un groupe lieur choisi dans un groupe constitué de phényle, vinyle, benzyle et alkyle ; et
(d) un groupe terminal azlactone contenant R₁ et R₂, dans lequel R₁ et R₂ sont chacun indépendamment choisis dans un groupe constitué d'hydrogène, alkyle, et aryle.

2. Polymère bifonctionnel selon la revendication 1, dans lequel les un ou plusieurs premiers polymères sont un copolymère avec un deuxième polymère qui est choisi dans un groupe constitué des polylysine, polyoxazoline, poly(méthacrylate de méthyle), poly-N-isopropylacrylamide, polydopamine, polyalcane, et polymère de glycine N-substitué.

3. Substrat revêtu comprenant :
(a) un substrat ; et
(b) une couche de polymère qui comprend un ou plusieurs premiers polymères, un ou plusieurs groupes azlactone fonctionnels liés à une première extrémité de chacun des un ou plusieurs premiers polymères, et un ou plusieurs groupes azide liés à une deuxième extrémité de chacun des un ou plusieurs premiers polymères, dans lequel les un ou plusieurs groupes azide lient la couche de polymère au substrat.

4. Substrat revêtu selon la revendication 3, le substrat étant choisi dans un groupe constitué de verre, silice, plastique, carbone, métal et oxyde métallique.

5. Substrat revêtu selon la revendication 3, dans lequel la couche de polymère comprend un polymère linéaire, un polymère multibras, un polymère en brosse, ou des nanoparticules.

6. Substrat revêtu selon la revendication 3, dans lequel les un ou plusieurs premiers polymères comprennent le polyéthylène glycol ou un polysaccharide.

7. Substrat revêtu selon la revendication 6, dans lequel les un ou plusieurs premiers polymères sont un copolymère avec un deuxième polymère qui est choisi dans un groupe constitué de polylysine, polyoxazoline, poly(méthacrylate de méthyle), poly-N-isopropylacrylamide, polydopamine, polyalcane et polymère glycine N-substitué.

8. Microréseau comprenant :
une pluralité de régions discrètes qui comprennent chacune un substrat revêtu, le substrat revêtu comprenant :
(a) un substrat ; et
(b) une couche de polymère qui comprend un ou plusieurs polymères, un ou plusieurs groupes azlactone fonctionnels liés à une première extrémité de chacun des un ou plusieurs polymères, et un ou plusieurs groupes azide liés à une deuxième extrémité de chacun des un ou plusieurs polymères, dans lequel les un ou plusieurs groupes azide lient la couche de polymère au substrat.

9. Procédé de préparation d'un polymère bifonctionnel, le procédé comprenant :
(a) la fourniture d'un polymère, dans lequel le polymère contient un ou plusieurs groupes acide carboxylique ou groupes ester activé liés à une première extrémité du polymère et un ou plusieurs groupes azide liés à une deuxième extrémité du polymère ;
(b) conversion d'au moins un groupe acide carboxylique ou groupe ester activé en un groupe halogénure d'aryle ou vinyle ; et
(c) liaison de vinyldialkylazlactone au groupe halogénure d'aryle ou vinyle.

10. Procédé selon la revendication 9, dans lequel le polymère est le polyéthylène glycol.

11. Procédé selon la revendication 9, dans lequel la vinyldialkyl-azlactone est lié par l'intermédiaire d'une réaction de couplage en utilisant un catalyseur au palladium, un ou plusieurs solvants, et une base.

12. Procédé selon la revendication 11, dans lequel le catalyseur au palladium est choisi dans un groupe constitué de quinoléine-8-carboxylate de palladium (II), chlorure de palladium (II), bromure de palladium (II), acétate de palladium (II), acétoacétate de palladium (II), bis(dibenzylidèneacétone)palladium(0), tris(dibenzylidèneacétone)dipalladium(0), trifluoroacétate de palladium (II), dimère de chlorure d'allylpalladium (II), dichlorure de bis(triphénylphosphine)palladium(II), dichlorobis(tricyclohexylphosphine)palladium(II) et
[1,1'-bis(di-tert-butylphosphino)ferrocène]dichloropalladium(II).

13. Procédé selon la revendication 11, dans lequel les un ou plusieurs solvants sont choisis dans un groupe constitué des diméthylformamide, diméthylsulfoxyde, toluène, tétrahydrofurane, dioxane, dichlorométhane, acétonitrile et alcool.

14. Procédé selon la revendication 11, dans lequel la base est choisie dans un groupe constitué des carbonate de potassium, carbonate de sodium, triéthylamine, N,N-diisopropyléthylamine, 4-(diméthylamino)pyridine, tert-butoxyde de potassium et tert-butoxyde de sodium.

15. Procédé de liaison d'un polymère bifonctionnel à un substrat, le procédé comprenant :
(a) la fourniture d'un substrat contenant des groupes fonctionnels alcyne ;
(b) fourniture d'un mélange comprenant le polymère bifonctionnel, un ou plusieurs solvants, un catalyseur, une base, et un agent réducteur ; et
(c) la mise en contact du substrat avec le mélange ; dans lequel le polymère bifonctionnel comprend un polymère, un ou plusieurs groupes azlactone fonctionnels liés à une première extrémité du polymère, et un ou plusieurs groupes azide liés à une deuxième extrémité du polymère.

16. Procédé selon la revendication 15, dans lequel le polymère est le polyéthylène glycol.

17. Procédé selon la revendication 15, dans lequel les un ou plusieurs solvants est choisi dans un groupe constitué des diméthylformamide, diméthylsulfoxyde, toluène, tétrahydrofurane, dioxane, acétonitrile et eau.

18. Procédé selon la revendication 15, dans lequel le catalyseur comprend un sel de cuivre (II) ou un sel de cuivre (I).

19. Procédé selon la revendication 18, dans lequel le catalyseur est choisi dans un groupe constitué des sulfate de cuivre, bromure de cuivre et iodure de cuivre.

20. Procédé selon la revendication 15, dans lequel le catalyseur comprend un catalyseur au ruthénium.

21. Procédé selon la revendication 20, dans lequel le catalyseur est choisi dans un groupe constitué du chlorure de pentaméthylcyclopentadiénylbis-(triphénylphosphine)ruthénium(II), chlorure de pentaméthylcyclopentadiényl-(cyclooctadiényl)ruthénium(II) et chlorure de pentaméthylcyclopentadiényl(norbornadiène)ruthénium(II) chlorure.

22. Procédé selon la revendication 15, dans lequel la base est choisie dans un groupe constitué des triéthylamine, N,N-diisopropyléthylamine, 4-(diméthylamino)pyridine, pyridine, quinolone, phénanthroline et imidazole.

23. Procédé selon la revendication 15, dans lequel l'agent réducteur est choisi dans un groupe constitué des ascorbate de sodium, tris(triazole)amine et hydroquinones.

24. Procédé d'isolement de biomolécules d'intérêt, le procédé comprenant :
(a) la fourniture d'un substrat fonctionnalisé, le substrat fonctionnalisé comprenant un substrat, un ou plusieurs polymères, un ou plusieurs groupes azlactone liés à une première extrémité de chacun des un ou plusieurs polymères, et un ou plusieurs groupes azide liés à une deuxième extrémité de chacun des un ou plusieurs polymères, dans lequel les un ou plusieurs groupes azide lient chacun des un ou plusieurs polymères au substrat ;
(b) la fourniture d'une solution aqueuse, dans lequel la solution aqueuse contient les biomolécules d'intérêt ; et
(c) la mise en contact du substrat fonctionnalisé avec la solution aqueuse pendant une période, dans lequel, pendant la période, les biomolécules d'intérêt se lient aux groupes azlactone.

25. Procédé selon la revendication 24, dans lequel la solution aqueuse comprend en outre un additif, dans lequel l'additif est choisi dans un groupe constitué des glycérol, oligoéthylène glycol, polyéthylène glycol, tensioactifs, alcool polyvinylique, sucres, solvants organiques et sels inorganiques.

26. Procédé selon la revendication 24, dans lequel un niveau de pH de la solution aqueuse est dans une plage d'environ 2 à environ 10.

27. Procédé selon la revendication 24, dans lequel la mise en contact du substrat fonctionnalisé avec la solution aqueuse est effectuée par impression par jet, impression à aiguilles, impression à la racle, impression au laser biologique, fluidique à base de capillaire ou immersion.
